(19) Europäisches Patentamt
European Patent Office
Office européen des brevets

(11) **EP 2 443 081 B1**

(12) **EUROPÄISCHE PATENTSCHRIFT**

(45) Veröffentlichungstag und Bekanntmachung des Hinweises auf die Patenterteilung:
**18.09.2013 Patentblatt 2013/38**

(21) Anmeldenummer: **10724860.1**

(22) Anmeldetag: **17.06.2010**

(51) Int Cl.:
*C07C 29/70* (2006.01)       *C07C 31/30* (2006.01)
*C07C 31/32* (2006.01)       *C07F 3/02* (2006.01)
*C07F 5/06* (2006.01)       *C08F 10/00* (2006.01)

(86) Internationale Anmeldenummer:
**PCT/EP2010/058550**

(87) Internationale Veröffentlichungsnummer:
**WO 2010/146122 (23.12.2010 Gazette 2010/51)**

(54) **KONZENTRIERTE LÖSUNGEN VON ERDALKALIMETALLALKOXIDEN IN APROTISCHEN LÖSUNGSMITTELN UND VERFAHREN ZU DEREN HERSTELLUNG**

CONCENTRATED SOLUTIONS OF ALKALINE-EARTH METAL OXIDES IN APROTIC SOLVENTS AND METHOD FOR THE PRODUCTION THEREOF

SOLUTIONS CONCENTRÉES D'ALCOXYDES DE MÉTAUX ALCALINOTERREUX DANS DES SOLVANTS APROTIQUES ET LEUR PROCÉDÉ DE PRODUCTION

(84) Benannte Vertragsstaaten:
**AL AT BE BG CH CY CZ DE DK EE ES FI FR GB GR HR HU IE IS IT LI LT LU LV MC MK MT NL NO PL PT RO SE SI SK SM TR**

(30) Priorität: **18.06.2009 DE 102009027018**

(43) Veröffentlichungstag der Anmeldung:
**25.04.2012 Patentblatt 2012/17**

(73) Patentinhaber: **Chemetall GmbH**
**60487 Frankfurt (DE)**

(72) Erfinder:
• **WIETELMANN, Ulrich**
**61381 Friedrichsdorf (DE)**
• **EMMEL, Ute**
**65929 Frankfurt (DE)**
• **RÖDER, Jens**
**38640 Goslar (DE)**
• **STEINBILD, Martin**
**60322 Frankfurt (DE)**
• **PAPSTEIN, Kay**
**38640 Goslar (DE)**

(74) Vertreter: **Rottmayer, Hans et al**
**Patente, Marken & Lizenzen**
**c/o Chemetall GmbH**
**Trakehner Strasse 3**
**60487 Frankfurt (DE)**

(56) Entgegenhaltungen:
**WO-A1-2007/026016       US-A- 4 748 283**

EP 2 443 081 B1

**Beschreibung**

Die Erfindung betrifft konzentrierte Lösungen von Erdalkalimetallalkoxiden in aprotischen Lösungsmitteln sowie ein Verfahren zu deren Herstellung. Magnesiumalkoxide werden unter anderem zur Herstellung geträgerter OlefinPolymerisationskatalysatoren vom Ziegler-Natta - Typ benötigt. Dazu werden gemäß dem Dokument EP 1031580 beispielsweise unlösliche Alkoxide wie zum Beispiel Magnesiumethoxid in Form sphärischer Partikel eingesetzt, welche durch Reaktion mit Titanchlorid oder einer anderen Titan-Halogenbindungen aufweisenden Verbindung z. B. Dicyclopentadienyl-titandichlorid ($Cp_2TiCl_2$) in die aktive Form überführt werden:

$$Mg(OEt)_2 \ + \ Cp_2TiCl_2 \ \longrightarrow \ Mg(OEt)_{2-x}Cl_x \ + \ Cp_2TiCl_{2-x}(OEt)_x$$

$$(x = 0 \ bis \ 2)$$

Eine weitere Möglichkeit, geträgerte Ziegler- Natta- Katalysatoren herzustellen besteht gemäß WO 85/02176 darin, von löslichen Magnesiumalkoxiden auszugehen. Während die meisten Magnesiumalkoholate wie z.B. die Mg-Salze des Methanols, Ethanols, Propanols, IsopropanoLs, tert- Butanols etc in aprotischen Lösungsmitteln unlöslich sind, erweisen sich die Mg- Verbindungen primärer Alkohole, die eine Verzweigung in 2- Stellung aufweisen, als löslich in Kohlenwasserstoffen. So sollen sich beispielsweise die Magnesiumsalze des 2- Methyl- 1- pentanols oder des 2- Ethyl- 1- hexanols in Konzentrationen von 1, 3 mol/l in Cyclohexan lösen. Auch gemischte Mgalkoxide, d.h. solche mit zwei verschiedenen Alkoxid- Resten Mg $(OR^1)$ $(OR^2)$ können gemäß WO 85/02176 kohlenwasserstoff- löslich sein, wenn der korrespondierende Alkohol $R^1OH$ ein in 2- Stellung verzweigter primärer Alkohol und der korrespondierende Alkohol $R^2OH$ ein sekundärer Alkohol ist.

Nachteilig an Kohlenwasserstoff- Lösungen, die außer Magnesium kein weiteres gelöstes Metall aufweisen, sind die relativ hohen Viskositäten. Weiterhin ist es nicht möglich, solche Lösungen direkt durch Umsetzung von Magnesiummetall mit dem Alkohol im gewünschten Kohlenwasserstoff herzustellen, ohne störende Hilfsmittel zuzugeben. Um eine direkte Umsetzung überhaupt zu ermöglichen, muß das Magnesiummetall aktiviert werden, was durch Anätzen mit iod erreicht werden kann. Aber auch mit dieser Maßnahme ist die Umsetzungsgeschwindigkeit selbst beim Einsatz von hochreaktivem Mg- Pulver noch sehr gering. So wird in EP 0156512 die Herstellung einer verdünnten Lösung von Magnesium- di (2- ethylhexoxid) , (MEHO) in Dodecan unter Verwendung von Iod beschrieben. Bei einer Reaktionstemperatur von 145 °C wird eine zehnstündige Reaktionszeit benötigt und das Produkt in Form einer viskosen Lösung erhalten. Eine andere Möglichkeit der Magnesiumaktivierung besteht gemäß WO 2007/026016 darin, das Erdalkalimetall mit Trialkylaluminiumverbindungen zu behandeln. Diese Methode hat den Vorteil, dass das Produkt nicht mit Iod verunreinigt wird. Allerdings sind die Reaktionsgeschwindigkeiten nicht zufriedenstellend und es werden viskose Produkte erhalten, welche relativ stark mit protischen Verunreinigungen, vor allem freiem Alkohol, verunreinigt sind.

Um die extrem langen Reaktionszeiten zu umgehen, werden Magnesiumalkoholatlösungen deshalb im Allgemeinen ausgehend von kommerziell erhältlichen Dialkylmagnesiumverbindungen hergestellt. Diese Syntheseroute hat jedoch den Nachteil, dass eine relativ teure Magnesiumquelle verwendet wird. Weiterhin impliziert sie eine Festlegung auf bestimmte Lösungsmittel, nämlich gesättigte Kohlenwasserstoffe: Dialkylmagnesiumverbindungen, beispielsweise Dibutylmagnesium, Butylethylmagnesium und Butyloctylmagnesium sind nämlich nur in gesättigten Kohlenwasserstoffen wie Hexan oder Heptan kommerziell erhältlich. Außerdem entstehen bei der Alkoholyse gem.

$$R^3R^4Mg + 2 \ ROH \rightarrow Mg(OR)_2 + R^3H + R^4H$$

unvermeidbar gesättigte Kohlenwasserstoffe $R^3H$ und $R^4H$, beispielsweise Butan oder Octan. Eine direkte Herstellung von Magnesiumalkoholaten in rein-aromatischen Lösungsmitteln wie Toluol oder Ethylbenzol ist deshalb über die Dialkylmagnesiumroute nicht möglich.

Eine weitere Synthesevariante zur Herstellung löslicher Erdalkalialkoholate besteht in der Umalkoholisierung unlöslicher Erdalkali- Alkoholate, hergestellt aus leicht flüchtigen Alkoholen (beispielsweise Ethanol) mit einem höher siedenden Alkohol, z.B.:

$$Mg \ (OR^5)_2 + 2 \ ROH \rightarrow Mg \ (OR)_2 + 2R^5OH$$

Nachteilig ist der relativ hohe, kostenintensive Aufwand dieser Methode: das Alkoholat $Mg(OR^5)_2$ muss zunächst aus dem flüchtigem Alkohol $R^5OH$ und Magnesiummetall hergestellt und isoliert werden, dann mit einem weniger flüchtigen Alkohol, beispielsweise 2-Ethylhexanol, umgesetzt werden und dann muss der flüchtigere Alkohol $R^5OH$ z.B. destillativ entfernt werden.

Die relativ hohe Viskosität von Magnesiumalkoxidlösungen wird durch Assoziationsphänomene verursacht. Aus dem Patent US 6,734,134 ist bekannt, dass die Viskosität durch Zugabe von Alkylaluminiumverbindungen reduziert werden kann. Das bevorzugte Verhältnis zwischen Alkylaluminiumverbindung und Mg-Alkoholat liegt zwischen 0,001: 1 und 1:1, mehr bevorzugt 0,01 bis 0,1:1 und ganz besonders bevorzugt 0,03 bis 0,05:1.

Es ist ein einfaches Verfahren gesucht, welches ausgehend von einer billigen Magnesiumquelle und mit hoher Raum/Zeit-Ausbeute wenig viskose, konzentrierte Lösungen eines Magnesiumalkoholates in aprotischen, bevorzugt aliphatischen oder aromatischen Kohlenwasserstofflösungsmitteln liefert. Weiterhin sollen die gewünschten Produkte möglichst geringe Gehalte an störenden Verunreinigungen wie z.B. Iod und protischen Stoffen wie Wasser und freien Alkoholen aufweisen, so dass sie für die Herstellung von Ziegler-Natta-Katalysatoren geeignet sind.

Die Aufgabe wird dadurch gelöst, dass gemischte Erdalkalialkoxidverbindungen $M(OCH_2R^6)_{2-x}(OR^7)_x$ in Mischung mit einer Aluminiumverbindung $Al(OCH_2R^6)_{3-y}(OR^7)_y$ in aprotischen Lösungsmitteln bereitgestellt werden. Dabei ist

- M ein Erdalkalimetall ausgewählt aus Mg, Ca, Ba, Sr;
- $OCH_2R^6$ ein Alkoxidrest bestehend aus mindestens 3 und höchstens 40 C-Atomen mit einer Verzweigung in 2-Position bezogen auf die O-Funktion, also $R^6 = -CHR^8R^9$ mit $R^8$, $R^9$ = unabhängig voneinander Alkylreste $C_1 - C_{18}$;
- $R^7$ ein Alkylrest mit 2- 15 C- Atomen, der entweder linear ist oder eine Verzweigung $\geq$ der 3- Position, bezogen auf die Stellung des Sauerstoff (O- Funktion) , aufweist
- und die Summe von x und y eine Zahl zwischen 0,01 und 0,8, bevorzugt 0,02 und 0,3 und besonders bevorzugt 0,03 und 0,2.

Das aprotische Lösungsmittel ist oder enthält entweder einen oder mehrere aliphatische Verbindungen mit 5 bis 20 C-Atomen, wobei sowohl zyklische als auch offenkettige Verbindungen möglich sind. Bevorzugt sind: Cyclohexan, Methylcyclohexan, Hexan, Heptan, Octan, Nonan, Dekan, Dodecan, Dekalin sowie handelsübliche Siedeschnitte wie Benzinfraktionen.

Das aprotische Lösungsmittel kann weiterhin Aromaten enthalten oder daraus bestehen. Bevorzugt sind: Benzol, Toluol, Ethylbenzol, Xylole sowie Cumol.

In einer weiteren Ausführungsform der Erfindung kann die erfindungsgemäße Erdalkalialkoxidlösung noch polare, aprotische Lösungsmittel wie z.B. Ether oder tertiäre Amine enthalten.

Der in 2- Position verzweigte Alkohol $(HOCH_2R^6)$ ist besonders bevorzugt ausgewählt aus der Gruppe bestehend aus: Isobutanol, 2- Methyl- 1- pentanol, 2- Ethyl- 1- butanol, 2- Ethyl- 1- pentanol, 2- Ethyl- 4- methyl- 1- pentanol, 2- Propyl- 1- heptanol, 2- Methyl- 1- hexanol, 2- Ethylhexanol und 2- Ethyl- 5- methyl- 1- octanol oder einer beliebigen Mischung aus mindestens zwei der aufgeführten Alkohole. Der primäre Alkohol $(HOR^7)$ ist bevorzugt ausgewählt aus der Gruppe bestehend aus: Ethanol, Propanol, Butanol, Pentanol, Hexanol, Octanol, Decanol, Dodekanol, 3- Methylbutan- 1- o1 oder einer beliebigen Mischung aus mindestens zwei der aufgeführten Alkohole.

Die erfindungsgemäßen Produkte werden im allgemeinen wie folgt hergestellt: handelsübliches Erdalkalimetall, bevorzugt Magnesiummetall, dieses bevorzugt in Pulver-, Granulat- oder Spanform, wird in einem wasserfreien aprotischen Lösungsmittel, bevorzugt aromatischen oder aliphatischen Kohlenwasserstoffen in einem inertisierten, d.h. getrockneten und mit Schutzgas wie Stickstoff oder Argon versehenem Rührwerksbehälter vorgelegt. Dann wird eine Alkylaluminiumverbindung, z.B. Trialkylaluminium wie Triethylaluminium, Tributylaluminium, ein Alkylaluminiumhydrid wie Dibutylaluminiumhydrid, ein Alkylaluminiumhalogenid wie Dibutylaluminiumchlorid oder eine Alkoxyaluminiumverbindung wie beispielsweise Diethylaluminiumethoxid zugegeben und 5 Minuten bis zwei Stunden bei 20 bis 180 °C, bevorzugt 40 bis 120 °C gerührt. Die optimale Menge an Alkylaluminiumverbindung richtet sich nach der Erdalkalimetallqualität, insbesondere der Magnesiumqualität, sowie der im nächsten Schritt zugegebenen Menge an Alkoholen. Im Allgemeinen liegt das Molverhältnis Alkylaluminiumverbindung zu den Alkoholen zwischen 0,0001 und 0,1 zu 1, bevorzugt zwischen 0,001 und 0,07 zu 1. Dann wird ein verzweiger Alkohol $HOCH_2R^6$ sowie ein unverzweigter oder eine Verzweigung $\geq$ der 3-Position aufweisender primärer Alkohol mit 2-15 C-Atomen $HOR^7$ zugegeben. Die Zugabe kann entweder nacheinander in beliebiger Reihenfolge oder in Mischung erfolgen. Bevorzugt wird zunächst der primäre Alkohol $R^7OH$ zugegeben, dann erst der verzweigte $HOCH_2R^6$. Die Zugabe kann bei Temperaturen zwischen 0 und 180°C erfolgen, bevorzugt zwischen 40 und 140 °C. Ganz besonders bevorzugt erfolgt sie am Siedepunkt des verwendeten Lösungsmittels, also z.B. im Falle von Toluol bei etwa 110°C. Die Reaktionszeit richtet sich nach der Reaktivität des verwendeten Erdalkalimetalls, insbesondere des Magnesiums, sowie des verwendeten Alkohols, dem stöchiometrischen Verhältnis zwischen Erdalkalimetall, insbesondere Magnesium, und den Alkoholen und der Reaktionstemperatur sowie nach den Anforderungen an das Endprodukt, insbesondere dem zulässigen Restgehalt an freiem Alkohol. Wird das Erdalkalimetall, insbesondere das Magnesium, im Überschuss eingesetzt, bevorzugt 1 bis 300 %, besonders bevorzugt 10 bis 100 %, reichen bei der Rückflussfahrweise 1 bis 6 Stunden Reaktionszeit aus.

Nach Beendigung der Reaktion, erkennbar am Versiegen des Wasserstoffstromes, wird gegebenenfalls überschüssiges Erdalkalimetall, insbesondere Magnesiummetall, von der gewünschten Produktlösung abgetrennt. Dies kann dekantativ, per Filtration oder Zentrifugation erfolgen.

Die nach erfindungsgemäßem Verfahren hergestellten Produkte sind abhängig von der Konzentration wenig viskos und sie enthalten wenig Wasser und freien Alkohol. Die Erdalkalimetall - Konzentrationen liegen bevorzugt im Bereich von ca. 0,4 bis 1,2 mmol/g, besonders bevorzugt zwischen 0,5 und 0,8 mmol/g. Die bei Raumtemperatur gemessenen Viskositäten liegen im Allgemeinen unter 500 mPa s, bevorzugt unter 250 mPa s, besonders bevorzugt unter 200 mPa s. Die Gehalte an protischen Verunreinigungen liegen, bezogen auf gelöstes Erdalkalielement, im Allgemeinen zwischen 0,1 und 15 mol%, bevorzugt 1 und 10 mol%, bezogen auf den Erdalkaligehalt.

Der Gehalt an gelöstem Aluminium liegt bezogen auf gelöstes Erdalkalimetall im Bereich zwischen 0,5 und 15 mol %, bevorzugt zwischen 1 und 8 mol%. Der Anteil des unverzweigten oder eine Verzweigung $\geq$ der 3-Position aufweisenden primären Alkohols mit 2-15 C-Atomen HOR[7] an der gesamten Alkoholmenge liegt zwischen 0,5 und 40 mol%, bevorzugt zwischen 1 und 20 mol%, besonders bevorzugt 1,5 und 10 mol%.

Die erfindungsgemäßen Produkte werden für die Herstellung von Polymerisationskatalysatoren, insbesondere heterogenisierte Polyolefinkatalysatoren vom Ziegler-Natta-Typ verwendet. Ferner können Sie in der organischen Synthese, beispielsweise als Basen eingesetzt werden.

### Beispiele

Alle Umsetzungen wurden in trockenen, mit Argon inertisierten Glasapparaturen vorgenommen. Es wurden handelsübliche Magnesiumspäne mit einem Überschuß von 100 %, bezogen auf den eingesetzten Alkohol, verwendet. Das Magnesium wurde wie in WO 2007/026016A1 beschrieben, generell mit ca. 2 mol% einer Trialkylaluminiumverbindung, bezogen auf eingesetzten Alkohol, aktiviert, d.h. reaktionsbereit gemacht.

**Vergleichsbeispiel 1**: Herstellung von Magnesium-2-ethylhexanolat in n-Heptan ohne Zusatz eines primären unverzweigten oder eine Verzweigung $\geq$ der 3-Position aufweisenden primären Alkohols mit 2-15 C-Atomen HOR[7] wie in WO 2007/026016A1 beschrieben

In einem 0,5 L-Doppelmantelglasreaktor mit Rückflusskühler und Tropftrichter wurden 14,9 g Magnesiumspäne und 437 g n-Heptan vorgelegt. Dann wurden 6,5 g einer 33%igen Lösung von Tributylaluminium in Cyclohexan zugespritzt. Dann wurde zum Siedepunkt aufgeheizt und 79,8 g 2-Ethylhexanol innerhalb von 90 Minuten zugetropft. Es entwickelten sich 1,04 L Gas. Nach Dosierende wurde der Reaktorinhalt noch weitere 225 min refluxiert, wobei sich weitere 3,1 L Gas entwickelten. Gegen Ende der Nachreaktionszeit wurde ein starkes Schäumen der Reaktionsmischung beobachtet.

Nach Abkühlung auf ca. 80 °C wurde die dunkelgraue Suspension filtriert. Es wurden 510 g einer viskosen Flüssigkeit mit einem Magnesiumgehalt von 0,28 mmol/g, entsprechend einem Umsatz von 49 % der theoretischen Ausbeute (d. Th.) erhalten. Die Produktlösung enthielt weiterhin 0,025 mmol/g Aluminium und sie wies einen Gehalt an protischen Verunreinigungen von 0,803 mmol/g auf.

**Beispiel 1**: Herstellung von Magnesium-2-ethylhexanolat in n-Heptan in Gegenwart von 1 mol% n-Octanol

In einem 0,5 L-Doppelmantelglasreaktor mit Rückflusskühler und Tropftrichter wurden 13,5 g Magnesiumspäne und 300 g n-Heptan vorgelegt. Dann wurden 4,8 g einer 25%igen Lösung von Triethylaluminium in Toluol zugespritzt. Dann wurde zum Siedepunkt aufgeheizt und ein Gemisch aus 73,0 g 2-Ethylhexanol und 0,73 g n-Octanol innerhalb von 150 Minuten zugetropft. Es entwickelten sich 4,9 L Gas. Nach Dosierende wurde der Reaktorinhalt noch weitere 190 Minuten refluxiert, wobei sich weitere 2,0 L Gas entwickelten. Es wurde kaum Schaumbildung beobachtet.

Nach Abkühlung auf ca. 80 °C wurde die dunkelgraue Suspension filtriert. Es wurden 363 g einer viskosen Flüssigkeit mit einem Magnesiumgehalt von 0,66 mmol/g, entsprechend einem Umsatz von 96 % d. Th., erhalten. Die Produktlösung enthielt weiterhin 0,027 mmol/g Aluminium und sie wies einen Gehalt an protischen Verunreinigungen von 0,108 mmol/g auf.

**Beispiel 2**: Herstellung von Magnesium-2-ethylhexanolat in n-Heptan in Gegenwart von 6 mol% Ethanol

In einem 0,5 L-Doppelmantelglasreaktor mit Rückflusskühler und Tropftrichter wurden 13,5 g Magnesiumspäne und 300 g n-Heptan vorgelegt. Dann wurden 4,8 g einer 25%igen Lösung von Triethylaluminium in Toluol zugespritzt. Dann wurde zum Siedepunkt aufgeheizt und ein Gemisch aus 73,0 g 2-Ethylhexanol und 1,46 g Ethanol innerhalb von 135 Minuten zugetropft. Es entwickelten sich 6,0 L Gas. Nach Dosierende wurde der Reaktorinhalt noch weitere 105 Minuten refluxiert, wobei sich weitere 0,55 L Gas ohne Aufschäumen entwickelten.

4

Nach Abkühlung auf ca. 80 °C wurde die dunkelgraue Suspension filtriert. Es wurden 377 g einer fast klaren Flüssigkeit mit einem Magnesiumgehalt von 0,72 mmol/g, entsprechend einem Umsatz von 99 % d. Th., erhalten. Die Produktlösung enthielt weiterhin 0,028 mmol/g Aluminium und sie wies einen Gehalt an protischen Verunreinigungen von 0,046 mmol/g auf.

**Vergleichsbeispiel 2**: Herstellung von Magnesium-2-ethylhexanolat in Toluol ohne Zusatz eines primären unverzweigten oder eine Verzweigung $\geq$ der 3-Position aufweisenden primären Alkohols mit 2-15 C-Atomen $HOR^7$ gemäß WO 2007/026016A1

In einem 1,0 L-Doppelmantelglasreaktor mit Rückflusskühler und Tropftrichter wurden 20,4 g Magnesiumspäne und 600 g Toluol vorgelegt. Dann wurden 9,0 g einer 21%igen Lösung von Triethylaluminium in Toluol zugespritzt, zum Siedepunkt aufgeheizt und 116 g 2-Ethylhexanol innerhalb von 52 Minuten zugetropft. Es entwickelten sich 3,25 L Gas. Nach Dosierende wurde der Reaktorinhalt noch weitere 900 Minuten refluxiert, wobei sich weitere 4,7 L Gas entwickelten. Es war eine sehr starke Schaumbildung zu beobachten.

Nach Abkühlung auf ca. 80 °C wurde die dunkelgraue Suspension filtriert. Es wurden 680 g einer viskosen Flüssigkeit mit einem Magnesiumgehalt von 0,50 mmol/g, entsprechend einem Umsatz von 87 % d. Th., erhalten. Die Produktlösung enthielt weiterhin 0,030 mmol/g Aluminium und sie wies einen Gehalt an protischen Verunreinigungen von 0,189 mmol/g auf.

**Beispiel 3**: Herstellung von Magnesium-2-ethylhexanolat in Toluol in Gegenwart von 8,7 mol% Ethanol

In einem 0,5 L-Doppelmantelglasreaktor mit Rückflusskühler und Tropftrichter wurden 20,3 g Magnesiumspäne und 450 g Toluol vorgelegt. Dann wurden 9,7 g einer 25%igen Lösung von Triethylaluminium in Toluol zugespritzt und zum Siedepunkt aufgeheizt. Dann wurden 3,39 g Ethanol und danach 109,7 g 2-Ethylhexanol innerhalb von 2 Stunden zugetropft. Es entwickelten sich 9,1 L Gas. Nach Dosierende wurde der Reaktorinhalt noch weitere 160 Minuten refluxiert, wobei sich weitere 0,84 L Gas ohne Aufschäumen entwickelten.

Nach Abkühlung auf ca. 80 °C wurde die Reaktionsmischung filtriert. Es wurden 565 g einer fast klaren Flüssigkeit mit einem Magnesiumgehalt von 0,73 mmol/g, entsprechend einem Umsatz von 100 % d. Th., erhalten. Die Produktlösung enthielt weiterhin 0,037 mmol/g Aluminium und sie wies einen Gehalt an protischen Verunreinigungen von 0,077 mmol/g auf.

Weitere Versuchsergebnisse werden in Tabelle 1 zusammengefasst:

Die Vergleichsbeispiele 1 und 2 erfolgten nach der technischen Lehre von WO 2007/026016A1, d.h. das Magnesium wurde mit Trialkylaluminiumlösungen aktiviert, und die Umsetzungen mit dem verzweigten Alkohol $HOCH_2R^6$ erfolgten am Siedepunkt.

In Heptan wird nach gut 5-stündiger Reaktionszeit in Abwesenheit eines primären unverzweigten oder eine Verzweigung $\geq$ der 3-Position aufweisenden primären Alkohols mit 2-15 C-Atomen $HOR^7$ nur etwa 50 % Umsetzung zum gewünschten Produkt Metallalkoholat beobachtet. Es wurde starkes Aufschäumen beobachtet. Das Verfahrensprodukt wies noch einen extrem hohen Gehalt an unerwünschten protischen Verunreinigungen auf: 0,803 mmol/g entsprechend 287 mol% bezogen auf gelöstes Magnesium. In Gegenwart von nur 1 mol % n-Octanol ließ sich in etwa derselben Zeit eine deutliche Steigerung des Reaktionsumsatzes auf 96 %, wie Beispiel 1 zeigt, erzielen. Demzufolge war der Gehalt an protischen Verunreinigungen ganz deutlich zurückgegangen; die Schaumbildung war gegenüber dem Vergleichsexperiment signifikant reduziert. Die Produktviskosität war trotz fast verdoppelter Metallalkoholat-Konzentration sogar leicht geringer.

Bei Erhöhung des Anteils an unverzweigtem oder eine Verzweigung $\geq$ der 3-Position aufweisenden primären Alkohols mit 2-15 C-Atomen $HOR^7$ auf 6 mol %, wie Beispiel 2 zeigt, wurde bereits nach vierstündiger Reaktionszeit ein fast quantitativer Umsatz beobachtet. Die erhaltene Lösung wies nur eine sehr geringe Konzentration an protischen Verbindungen in Form von Wasser und freien Alkoholen auf und die Lösung war nur sehr wenig viskos (195 mPa s).

Ähnliche Beobachtungen ließen sich auch bei Verwendung von aromatischen Lösungsmitteln, in Beispiel 3 und Vergleichsbeispiel 2 Toluol, machen. In Gegenwart von unverzweigtem oder eine Verzweigung $\geq$ der 3-Position aufweisendem primären Alkohol mit 2-15 C-Atomen $HOR^7$, siehe im Beispiel 3 Ethanol, war die Reaktionsgeschwindigkeit wesentlich erhöht und die Produktqualität verbessert.

Tabelle 1

| Versuchsbeispiel | Lösemittel | ROH (mol %) | Reaktionszeit (min) | Mg (mmol/g) | Protische Verunreinigungen (mmol/g)/ (mol%)* | Umsatz (% d. Th.) | Viskosität (mPa s) |
|---|---|---|---|---|---|---|---|
| 1 | n-Heptan | 1 (n-Octanol) | 340 | 0,66 | 0,108/ 16 | 96 | 830 |
| 2 | n-Heptan | 6 (Ethanol) | 240 | 0,72 | 0,046/ 6,4 | 99 | 195 |
| Vgl. 1 | n-Heptan | ./. | 315 | 0,28 | 0,803/ 287 | 49 | 850 |
| 3 | Toluol | 8,7 (Ethanol) | 280 | 0,73 | 0,077/ 11 | 101 | 100 |
| Vgl. 2 | Toluol | ./. | 952 | 0,50 | 0,189/ 38 | 87 | 540 |
| *bezogen auf gelöstes Magnesium | | | | | | | |

**Patentansprüche**

1. Lösungen von gemischten Erdalkalialkoxidverbindungen $M(OCH_2R^6)_{2-x}(OR^7)_x$ und einer Aluminiumverbindung $Al(OCH_2R^6)_{3-y}(OR^7)_y$ in aprotischen Lösungsmitteln wobei

   • M ein Erdalkalimetall ausgewählt aus Mg, Ca, Ba, Sr;
   • $OCH_2R^6$ ein Alkoxidrest bestehend aus mindestens 3 und höchstens 40 C-Atomen mit einer Verzweigung in 2-Position, bezogen auf die O-Funktion, also $R^6 = -CHR^8R^9$ mit $R^8$, $R^9$ = unabhängig voneinander Alkylreste $C_1 - C_{18}$;
   • $R^7$ ist ein Alkylrest mit 2-15 C-Atomen, der entweder linear ist oder eine Verzweigung $\geq$ der 3-Position, bezogen auf die O-Funktion, aufweist;
   • und die Summe von x und y eine Zahl zwischen 0,01 und 0,8, bevorzugt 0,02 und 0,3 und besonders bevorzugt 0,03 und 0,2 ist.

2. Lösungen nach Anspruch 1, **dadurch gekennzeichnet, dass** die Erdalkalimetallkonzentration bevorzugt im Bereich von 0,4 bis 1,2, besonders bevorzugt zwischen 0,5 bis 0,8 mmol/g liegt.

3. Lösungen nach Anspruch 1 oder 2, **dadurch gekennzeichnet, dass** die Raumtemperatur-Viskositäten im Allgemeinen unter 500 mPa s, bevorzugt unter 250, besonders bevorzugt unter 200 mPa s liegen.

4. Lösungen nach Anspruch 1 bis 3, **dadurch gekennzeichnet, dass** die Gehalte an protischen Verunreinigungen, bezogen auf gelöstes Erdalkalielement, im Allgemeinen zwischen 0,1 und 15 mol%, bevorzugt 1 und 10 mol% liegen.

5. Lösungen nach Anspruch 1 bis 4, **dadurch gekennzeichnet, dass** der Gehalt an gelöstem Aluminium bezogen auf gelöstes Erdalkalimetall im Bereich zwischen 0,5 und 15 mol %, bevorzugt zwischen 1 und 8 mol% liegt.

6. Verfahren zur Herstellung von gemischten Erdalkalialkoxidverbindungen $M(OCH_2R^6)_{2-x}(OR^7)_x$ in aprotischen Lösungsmitteln, **dadurch gekennzeichnet, dass** ein mittels Alkylaluminiumverbindungen aktiviertes Erdalkalimetall in einem aprotischen Lösungsmittel mit einem in 2-Stellung verzweigten primären Alkohol sowie einem unverzweigten oder eine Verzweigung $\geq$ der 3-Position aufweisenden primären Alkohol mit 2-15 C-Atomen $HOR^7$ umgesetzt wird.

7. Verfahren nach Anspruch 6, **dadurch gekennzeichnet, dass** als Alkylaluminiumverbindung Trialkyl-, Alkyl-Alkoxy- und/oder Alkyl-Halogenid-Verbindungen eingesetzt werden.

8. Verfahren nach Anspruch 6 oder 7, **dadurch gekennzeichnet, dass** als Lösemittel Kohlenwasserstoffe eingesetzt werden, wobei entweder aliphatische Lösemittel ausgewählt aus der Gruppe bestehend aus Cyclohexan, Methylcyclohexan, Hexan, Heptan, Octan, Nonan, Dekan, Dodecan, Dekalin sowie handelsübliche Siedeschnitte oder aromatische Lösemittel ausgewählt aus der Gruppe bestehend aus Benzol, Toluol, Ethylbenzol, Xylolen sowie Cumol verwendet werden.

9. Verfahren nach Anspruch 6 bis 8, **dadurch gekennzeichnet, dass** die Umsetzung bei Temperaturen zwischen etwa 0 und 180 °C erfolgt, bevorzugt zwischen etwa 40 und 140 °C, insbesondere bei der Siede-Temperatur des Lösemittels.

10. Verfahren nach Anspruch 6 bis 9, **dadurch gekennzeichnet, dass** als in 2-Stellung verzweigte Alkohole $(HOCH_2R^6)$ Isobutanol, 2-Methyl-1-pentanol, 2-Ethyl-1-butanol, 2-Ethyl-1-pentanol, 2-Ethyl-4-methyl-1-pentanol, 2-Propyl-1-heptanol, 2-Methyl-1-hexanol, 2-Ethylhexanol und 2-Ethyl-5-methyl-1-octanol oder eine beliebige Mischung aus mindestens zwei der aufgeführten Alkohole und als unverzweigte oder eine Verzweigung $\geq$ der 3-Position aufweisende primäre Alkohole mit 2-15 C-Atomen $(HOR^7)$ Ethanol, Propanol, Butanol, Pentanol, Hexanol, Octanol, Decanol, Dodekanol, 3-Methylbutan-1-ol oder eine beliebige Mischung aus mindestens zwei der aufgeführten Alkohole eingesetzt werden.

11. Verfahren nach Anspruch 6 bis 10, **dadurch gekennzeichnet, dass** zunächst der Alkohol $(HOR^7)$ zugegeben, dann erst der in 2-Stellung verzweigte Alkohol $(HOCH_2R^6)$ zugegeben wird.

12. Verwendung einer Lösung gemäß einem der Ansprüche 1 bis 5 zur Herstellung von Polymerisationskatalysatoren, insbesondere heterogenisierten Polyolefinkatalysatoren vom Ziegler-Natta-Typ.

13. Verwendung einer Lösung gemäß einem der Ansprüche 1 bis 5 in der organischen Synthese, beispielsweise als Base.

**Claims**

1. Solutions of mixed alkaline-earth alkoxide compounds M $(OCH_2R^6)_{2-x}(OR^7)_x$ and an aluminium compound Al $(OCH_2R^6)_{3-y}(OR^7)_y$ in aprotic solvents, wherein

   • M is an alkaline-earth metal selected from Mg, Ca, Ba, Sr;
   • $OCH_2R^6$ is an alkoxide radical consisting of at least 3 and at most 40 C-atoms having a branch in the 2-position, relative to the O-function, that is, $R^6$ = -$CHR^8R^9$ with $R^8$, $R^9$ = independently of each other alkyl radicals $C_1$ - $C_{18}$;
   • $R^7$ is an alkyl radical with 2-15 C-atoms which is either linear or has a branch $\geq$ the 3-position, relative to the O-function;
   • and the sum of x and y is a number between 0.01 and 0.8, preferably 0.02 and 0.3, and particularly preferably 0.03 and 0.2.

2. Solutions according to claim 1, **characterised in that** the alkaline-earth metal concentration preferably lies in the range of 0.4 to 1.2, particularly preferably between 0.5 to 0.8 mmol/g.

3. Solutions according to claim 1 or 2, **characterised in that** the room-temperature viscosities generally lie below 500 mPa s, preferably below 250, particularly preferably below 200 mPa s.

4. Solutions according to claim 1 to 3, **characterised in that** the content of protic impurities, relative to dissolved alkaline-earth element, generally lies between 0.1 and 15 mol%, preferably 1 and 10 mol%.

5. Solutions according to claim 1 to 4, **characterised in that** the content of dissolved aluminium, relative to dissolved alkaline-earth metal, lies in the range between 0.5 and 15 mol%, preferably between 1 and 8 mol%.

6. Method for preparing mixed alkaline-earth alkoxide compounds $M(OCH_2R^6)_{2-x}(OR^7)_x$ in aprotic solvents, **characterised in that** an alkaline-earth metal, activated by means of alkyl aluminium compounds, in an aprotic solvent is reacted with a primary alcohol which is branched in the 2-position and also with a primary alcohol $HOR^7$ which is unbranched or has a branch $\geq$ the 3-position with 2-15 C-atoms.

7. Method according to claim 6, **characterised in that** trialkyl, alkyl-alkoxy and/or alkyl halide compounds are used as the alkyl aluminium compound.

8. Method according to claim 6 or 7, **characterised in that** hydrocarbons are used as the solvents, wherein either aliphatic solvents, selected from the group consisting of cyclohexane, methylcyclohexane, hexane, heptane, octane, nonane, decane, dodecane, decalin and also commercially available boiling cuts, or aromatic solvents, selected from the group consisting of benzene, toluene, ethyl benzene, xylenes and also cumene, are used.

9. Method according to claim 6 to 8, **characterised in that** the reaction is effected at temperatures between approximately 0 and 180°C, preferably between approximately 40 and 140°C, in particular at the boiling temperature of the solvent.

10. Method according to claim 6 to 9, **characterised in that** as the alcohols $(HOCH_2R^6)$ that are branched in the 2-position isobutanol, 2-methyl-1-pentanol, 2-ethyl-1-butanol, 2-ethyl-1-pentanol, 2-ethyl-4-methyl-1-pentanol, 2-propyl-1-heptanol, 2-methyl-1-hexanol, 2-ethyl hexanol and 2-ethyl-5-methyl-1-octanol or any mixture of at least two of the listed alcohols is used, and as primary alcohols $(HOR^7)$ which are unbranched or have a branch $\geq$ the 3-position with 2-15 C-atoms ethanol, propanol, butanol, pentanol, hexanol, octanol, decanol, dodecanol, 3-methylbutan-1-ol or any mixture of at least two of the listed alcohols is used.

11. Method according to claim 6 to 10, **characterised in that** the alcohol $(HOR^7)$ is added first and only then is the alcohol $(HOCH_2R^6)$, which is branched in the 2-position, added.

12. Use of a solution according to one of claims 1 to 5 for preparing polymerization catalysts, in particular heterogenized polyolefin catalysts of the Ziegler-Natta type.

**13.** Use of a solution according to one of claims 1 to 5 in organic synthesis, for example as a base.

**Revendications**

**1.** Solutions de mélanges de composés de type alcoolate de métal alcalino-terreux, de formule $M(OCH_2R^6)_{2-x}(OR^7)_x$, et d'un composé de l'aluminium, de formule $Al(OCH_2R^6)_{3-y}(OR^7)_y$, dans des solvants aprotiques, étant entendu que :

- M représente un métal alcalino-terreux choisi parmi les magnésium, calcium, baryum et strontium ;
- $OCH_2R^6$ représente un reste alcoolate comprenant au moins 3 et au plus 40 atomes de carbone et doté d'une ramification en position 2 par rapport à l'atome d'oxygène fonctionnel, $R^6$ représentant donc un groupe de formule $-CHR^8R^9$ où $R^8$ et $R^9$ représentent chacun, indépendamment l'un de l'autre, un groupe alkyle en $C_1$-$C_{18}$ ;
- $R^7$ représente un groupe alkyle comportant de 2 à 15 atomes de carbone, qui est soit linéaire, soit doté d'une ramification en position 3 ou plus loin par rapport à l'atome d'oxygène fonctionnel ;
- et la somme des indices x et y est un nombre valant de 0,01 à 0,8, de préférence de 0,02 à 0,3 et surtout de 0,03 à 0,2.

**2.** Solutions conformes à la revendication 1, **caractérisées en ce que** la concentration de métal alcalino-terreux se situe de préférence dans l'intervalle allant de 0,4 à 1,2 mmol/g, et surtout dans l'intervalle allant de 0,5 à 0,8 mmol/g.

**3.** Solutions conformes à la revendication 1 ou 2, **caractérisées en ce que** leur viscosité à température ambiante est en général inférieure à 500 mPa.s, de préférence inférieure à 250 mPa.s, et surtout inférieure à 200 mPa.s.

**4.** Solutions conformes à l'une des revendications 1 à 3, **caractérisées en ce que** les teneurs en impuretés protiques, par rapport à l'élément alcalino-terreux dissous, valent en général de 0,1 à 15 % en moles, et de préférence de 1 à 10 % en moles.

**5.** Solutions conformes à l'une des revendications 1 à 4, **caractérisées en ce que** la teneur en aluminium dissous, par rapport au métal alcalino-terreux dissous, vaut de 0,5 à 15 % en moles, et de préférence de 1 à 8 % en moles.

**6.** Procédé de préparation de mélanges de composés de type alcoolate de métal alcalino-terreux, de formule $M(OCH_2R^6)_{2-x}(OR^7)_x$, dans des solvants aprotiques, **caractérisé en ce que** l'on fait réagir un métal alcalino-terreux activé au moyen de composés de type alkyl-aluminium, dans un solvant aprotique, avec un alcool primaire ramifié en position 2 ainsi qu'avec un alcool primaire de formule $HOR^7$, non ramifié ou porteur d'une ramification en position 3 ou plus loin, et comportant de 2 à 15 atomes de carbone.

**7.** Procédé conforme à la revendication 6, **caractérisé en ce que** l'on utilise, en tant que composés de type alkyl-aluminium, des composés de type trialkyl-aluminium, alkyl-alcoxy-aluminium et/ou halogénure d'alkyl-aluminium.

**8.** Procédé conforme à la revendication 6 ou 7, **caractérisé en ce que** l'on utilise des hydrocarbures comme solvants, c'est-à-dire que l'on utilise soit des solvants aliphatiques choisis dans l'ensemble constitué par les cyclohexane, méthyl-cyclohexane, hexane, heptane, octane, nonane, décane, dodécane et décaline ainsi que les fractions de distillation commercialisées, soit des solvants aromatiques choisis dans l'ensemble constitué par les benzène, toluène, éthyl-benzène, xylènes et cumène.

**9.** Procédé conforme à l'une des revendications 6 à 8, **caractérisé en ce que** la réaction est effectuée à des températures valant de 0 à 180 °C et de préférence de 40 à 140 °C, et en particulier à la température d'ébullition du solvant.

**10.** Procédé conforme à l'une des revendications 6 à 9, **caractérisé en ce que** l'on utilise, en tant qu'alcool ramifié en position 2 (de formule $HOCH_2R^6$), de l'isobutanol, du 2-méthyl-pentan-1-ol, du 2-éthyl-butan-1-ol, du 2-éthyl-pentan-1-ol, du 2-éthyl-4-méthyl-pentan-1-ol, du 2-propyl-heptan-1-ol, du 2-méthyl-hexan-1-ol, du 2-éthyl-hexanol ou du 2-éthyl-5-méthyl-octan-1-ol, ou un mélange quelconque d'au moins deux des alcools mentionnés, et en tant qu'alcool primaire non ramifié ou porteur d'une ramification en position 3 ou plus loin et comportant de 2 à 15 atomes de carbone (de formule $HOR^7$), de l'éthanol, du propanol, du butanol, du pentanol, de l'hexanol, de l'octanol, du décanol, du dodécanol ou du 3-méthyl-butan-1-ol, ou un mélange quelconque d'au moins deux des alcools mentionnés.

**11.** Procédé conforme à l'une des revendications 6 à 10, **caractérisé en ce que** l'on ajoute d'abord l'alcool de formule $HOR^7$, et ensuite seulement l'alcool ramifié en position 2, de formule $HOCH_2R^6$.

**12.** Utilisation d'une solution conforme à l'une des revendications 1 à 5, pour la préparation de catalyseurs de polymérisation, en particulier de catalyseurs de production de polyoléfines de type Ziegler-Natta, hétérogénéisés.

**13.** Utilisation d'une solution conforme à l'une des revendications 1 à 5 en synthèse organique, par exemple en tant que base.

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 1031580 A
- WO 8502176 A
- EP 0156512 A
- WO 2007026016 A
- US 6734134 B
- WO 2007026016 A1